# EUROPEAN PATENT APPLICATION

(11) **EP 4 091 673 A1**
(43) Date of publication of application: **23.11.2022**
(21) Application number: 20913307.3
(22) Date of filing: 11.12.2020
(51) Int. Cl.: A61P 43/00, C12P 1/04, A23L 33/10, A23L 33/135, C12N 1/20, A61K 35/745, A23L 2/52, A23L 2/38

(54) **ANTI-AGING COMPOSITION**

(30) Priority: 17.01.2020 JP 2020006097; 02.04.2020 JP 2020066742
(71) Applicant: Morinaga Milk Industry Co., Ltd., Minato-ku Tokyo 108-8384 (JP)
(72) Inventor: TODA, Kazuya, Zama-shi, Kanagawa 252-8583 (JP); ONO, Kazuya, Zama-shi, Kanagawa 252-8583 (JP); YOSHIMOTO, Shin, Zama-shi, Kanagawa 252-8583 (JP); MITSUYAMA, Eri, Zama-shi, Kanagawa 252-8583 (JP)
(74) Representative: Hoffmann Eitle
(86) International application number: PCT/JP2020/046210
(87) International publication number: WO 2021/145113

(57) **Abstract**

An object is to provide a composition for anti-aging. One, two or more kinds selected from *Bifidobacterium longum* NITE BP-02621, a culture of the bacterium and a treated product of the bacterium are comprised in an anti-aging composition. From the composition of the invention, an anti-aging effect can be preferably obtained through a cellular senescence inhibitory effect and/or a mitochondrial function-improving effect. Moreover, the composition of the invention can be preferably used for prolonging lifespan and/or for suppressing a decline in motor function.

## Description

### Technical Field

The present invention relates to a composition for anti-aging.

### Background Art

Many people wish for a long and healthy life. For this reason, various researches aiming at anti-aging have long been carried out.

One of the symptoms of aging is aging at the cellular level (cellular senescence).

Cellular senescence is generally defined as a state in which a cell maintains the viable state and has the metabolic activity but has lost the proliferative capacity. The number of times a mammalian cell can divide and proliferate is limited, and, when the number of cell divisions reaches the end of life (the limit), cellular senescence is induced. It is known that, when cells isolated from an aged individual and cells isolated from a young individual are cultured *in vitro* to compare the numbers of cell divisions, the cells isolated from the aged individual reach the division limit earlier. It is known that cellular senescence can be caused also by various stimuli or factors, such as telomere shortening due to replication of DNA ends, DNA damage, changes in the expression or the activities of tumor suppressor genes and oncogenes, oxidative stress, inflammation, chemotherapeutic agents and exposure to UV irradiation and ionizing radiation (NPL 1).

Various factors that are collectively called senescence associated secretory phenotype (SASP) are secreted from cellular senescence-induced cells (senescent cells) and cause aging of surrounding cells. SASP which has traveled through the whole body through the vessels promotes cellular senescence or oncogenesis at the site where the SASP has reached and causes chronic inflammation or migration of immune cells, and as a result, the functions of tissues are sometimes damaged (NPL 1). Cellular senescence is believed to be induced in various tissues with aging, is suggested to be relevant to arteriosclerosis, diabetes, dementia and the like and is believed to be one of the factors causing age-related diseases (NPL 2).

In view of such characteristics of senescent cells, prevention of accumulation of senescent cells (senolytics) has been attracting increasing attention, and elimination of senescent cells with a senolytic has been shown to be effective for some age-related diseases in animal research and human clinical trials (NPL 2). Moreover, in a mouse model, lifespan-prolonging effects of a senolytic have been shown, and anti-aging effects at the individual level have been elucidated (NPL 3). It has been thus suggested that cellular senescence plays an important role in the aging of individuals.

It is proposed that aging at the individual level is caused not only by accumulation of senescent cells but also caused when free radicals (reactive oxygen species and the like) that are produced mainly in mitochondria as by-products of energy metabolism oxidize DNA, proteins, lipids and the like and induce cytotoxicity (NPL 4). In fact, in mice with induced muscle mitochondrial dysfunction, systemic aging symptoms were promoted (NPL 5). It is believed that an increase in mitochondria with age-related dysfunction is one of the factors that cause excessive accumulation of free radicals. Excessive accumulation of free radicals is also well known as one of the stress types that cause cellular senescence. Accordingly, it is considered that a component which can improve mitochondrial function can be useful for anti-aging.

Many organisms have genes that are relevant to elimination of free radicals and repair of damage by oxidative stress such as free radicals. For example, superoxide dismutase (SOD) is a major oxidative stress defense enzyme which makes superoxide, which is one of reactive oxygen species (ROS), nontoxic. In fact, it has been reported that cellular senescence was promoted in mice without SOD1 expression (NPL 6).

### Citation List

### Non Patent Literature

NPL 1: Eiji Hara, "New era of cellular senescence", Experimental Medicine, 37(11), 1728-1733, (2019)
NPL 2: Paez-Ribes M, Gonzalez-Gualda E, Doherty GJ, Munoz-Espin D. EMBO Molecular Medicine, 11(12), PMID:31746100, (2019)
NPL 3: Baker DJ, Childs BG, Durik M, Wijers ME, Sieben CJ, Zhong JA, Saltness R, Jeganathan KB, Verzosa GC, Pezeshki A et al., Nature, 530, 184-189, (2016)
NPL 4: R Perez-Campo, M Lopez-Torres, S Cadenas, C Rojas, G Barja. J. Comp. Physiol. B, 168(3), 149-158, (1998)
NPL 5: Tezze C, Romanello V, Desbats MA, Cell Metab., 25(6), 1374-1389, (2017)
NPL 6: Yiqiang Zhang et al., Redox Biol, 11, 30-37, (2017)

### Summary of Invention

### Technical Problem

An object of the invention is to provide a composition for anti-aging.

### Solution to Problem

As a result of extensive research to achieve the object, the present inventors have found that *Bifidobacterium longum* NITE BP-02621, a culture thereof or a treated product thereof has an anti-aging effect and thus completed the invention.

That is, an embodiment of the invention is an anti-aging composition comprising one, two or more kinds selected from *Bifidobacterium longum* NITE BP-02621, a culture of the bacterium and a treated product of the bacterium.

In the embodiment, the anti-aging may be through a cellular senescence inhibitory effect and/or a mitochondrial function-improving effect.

The composition in the embodiment may be a composition which is used for prolonging lifespan and/or for suppressing a decline in motor function.

Another embodiment of the invention is a composition comprising one, two or more kinds selected from *Bifidobacterium longum* NITE BP-02621, a culture of the bacterium and a treated product of the bacterium which is used for anti-aging.

The composition of the invention is preferably a food or a drink.

The composition of the invention is preferably a pharmaceutical product.

Another embodiment of the invention is use of one, two or more kinds selected from *Bifidobacterium longum* NITE BP-02621, a culture of the bacterium and a treated product of the bacterium in the manufacture of an anti-aging composition.

Another embodiment of the invention is use of one, two or more kinds selected from *Bifidobacterium longum* NITE BP-02621, a culture of the bacterium and a treated product of the bacterium in anti-aging.

Another embodiment of the invention is an anti-aging method including administering one, two or more kinds selected from *Bifidobacterium longum* NITE BP-02621, a culture of the bacterium and a treated product of the bacterium to a subject.

### Advantageous Effects of Invention

According to the invention, an anti-aging effect can be obtained, for example, by inhibiting cellular senescence and/or improving mitochondrial function. Therefore, lifespan can be prolonged, and a decline in motor function can be suppressed.

### Brief Description of Drawings

[Fig. 1] A graph showing the SA-β-Gal staining rates (averages) of TIG-3 cells on the second day after cellular senescence induction.
[Fig. 2] A graph showing the relative expression levels of *p16* gene in TIG-3 cells on the third day after cellular senescence induction.
[Fig. 3] A graph showing the survival rates of nematodes.
[Fig. 4] A graph showing the survival rates of nematodes with induced mitochondrial dysfunction.

### Description of Embodiments

Next, the invention is explained in detail. However, the invention is not limited to the following embodiments and can be freely changed within the scope of the invention.

The composition of the invention comprises one, two or more kinds selected from *Bifidobacterium longum* NITE BP-02621, a culture of the bacterium and a treated product of the bacterium.

*Bifidobacterium longum* NITE BP-02621 was deposited for an international deposit under the Budapest Treaty on January 26, 2018 to NITE Patent Microorganisms Depositary (NPMD), National Institute of Technology and Evaluation (Room 122, 2-5-8 Kazusakamatari, Kisarazu-shi, Chiba 292-0818) and given an accession number of NITE BP-02621. *Bifidobacterium longum* NITE BP-02621 is also called "BB536". The identical bacterium, *Bifidobacterium longum* subsp. *longum* ATCC BAA-999 (number: ATCC BAA-999), can be acquired from American Type Culture Collection (ATCC; 10801 University Boulevard, Manassas, VA 20110, United States of America) as ATCC BAA-999 (see, for example, JP-A-2012-223134).

*Bifidobacterium longum* NITE BP-02621 is not limited to the strain deposited or registered at the certain organizations under the bacterial name itself (also called "deposited strain" below for the convenience of explanation) but includes substantially equivalent strains thereof (also called "derived strains" or "induced strains"). That is, *Bifidobacterium longum* NITE BP-02621 is not limited to the strain deposited to the depositary with the accession number itself but includes substantially equivalent strains thereof. The "substantially equivalent strains of the deposited strain" of the bacterium are strains which belong to the identical species as that of the deposited strain, have a genome sequence similarity (Average Nucleotide Identity value) of preferably 99.0% or more, more preferably 99.5% or more, further preferably 100% identity to the deposited strain and preferably have the identical bacteriological properties to those of the deposited strain. Examples of the substantially equivalent strains of the deposited strain of the bacterium may be strains derived from the deposited strain as the parent strain. The derived strains include a strain bred from the deposited strain and a strain naturally generated from the deposited strain. Breeding methods include modification by the genetic engineering technique and modification by mutagenesis. The mutagenesis includes X-ray irradiation, ultraviolet irradiation and treatment with a mutagen such as N-methyl-N'-nitro-N-nitrosoguanidine, ethyl methanesulfonate and methyl methanesulfonate. The strain naturally generated from the deposited strain is a strain which is naturally generated from the deposited strain during use. Such a strain is a mutant naturally generated during culture of the deposited strain (for example, passaging culture). The derived strains may be constructed with a kind of modification or constructed with two or more kinds of modification.

As *Bifidobacterium longum* NITE BP-02621 comprised in the composition of the invention, a commercial product may be used. Alternatively, one appropriately produced and obtained may be used, and one obtained by culturing *Bifidobacterium longum* NITE BP-02621 described above may also be used.

The culture method is not particularly restricted as long as *Bifidobacterium longum* NITE BP-02621 can grow. As the culture method, for example, a method which is generally used for culturing *Bifidobacterium longum* NITE BP-02621 can be used directly or with appropriate modification. The culture temperature may be, for example, 25 to 50°C and is preferably 35 to 42°C. The culture can be conducted preferably under anaerobic conditions and can be conducted, for example, while flowing an anaerobic gas such as carbon dioxide. The culture can also be conducted under microaerophilic conditions such as static liquid culture. The culture can be conducted, for example, until *Bifidobacterium longum* NITE BP-02621 grows to a desired degree.

The medium used for the culture is not particularly restricted as long as *Bifidobacterium longum* NITE BP-02621 can grow. As the medium, for example, a medium which is generally used for culturing *Bifidobacterium longum* NITE BP-02621 can be used directly or with appropriate modification. That is, as a carbon source, for example, saccharides such as galactose, glucose, fructose, mannose, cellobiose, maltose, lactose, sucrose, trehalose, starch, starch hydrolysate and molasses can be used depending on the assimilation properties. As a nitrogen source, for example, ammonia and ammonium salts and nitrates such as ammonium sulfate, ammonium chloride and ammonium nitrate can be used. Moreover, as an inorganic salt, for example, sodium chloride, potassium chloride, potassium phosphate, magnesium sulfate, calcium chloride, calcium nitrate, manganese chloride, ferrous sulfate and the like can be used. Furthermore, organic components such as peptone, soybean powder, a defatted soybean cake, meat extract and yeast extract may also be used. Specific examples of the medium which is generally used for culturing *Bifidobacterium longum* NITE BP-02621 include reinforced clostridial medium, MRS medium (de Man, Rogosa, and Sharpe medium), mMRS medium (modified MRS medium), TOSP medium (TOS propionate medium), TOSP Mup medium (TOS propionate mupirocin medium), GAM (Gifu Anaerobic Medium) medium, YCFA (Yeast Extract-casein Hydrolysate Acid) medium and the like.

The form of *Bifidobacterium longum* NITE BP-02621 comprised in the composition of the invention may be any of bacterial cells, a culture of the bacterium and a treated product of the bacterium.

The bacterial cells are generally preferably comprised in the form comprising living bacterial cells but are not particularly limited. The bacterial cells may be, for example, living bacterial cells, dead bacterial cells or a mixture of living bacterial cells and dead bacterial cells.

As the culture of the bacterium, for example, the culture obtained through culture may be used directly. Alternatively, a diluted or concentrated culture may be used, or bacterial cells collected from the culture may also be used. Moreover, as the culture, a culture supernatant or a culture fraction may also be used. When a culture supernatant is used, for example, a supernatant of a culture solution obtained by culturing in GAM medium under the conditions of 37°C and 16 h can be preferably used.

As the treated product of the bacterium, crushed, heated or lyophilized bacterial cells or culture, a diluted product thereof, a dried product thereof or a fraction thereof can be used.

The composition of the invention can exhibit an anti-aging effect. The "anti-aging" means to prevent or suppress an age-related change, generally an unfavorable change, or to delay the progress thereof.

The anti-aging effect that the composition of the invention exhibits may be through a cellular senescence inhibitory effect. The "inhibition of cellular senescence" includes inhibition of the change of normal cells into senescent cells. The "normal cells" mean cells which maintain the viable state and which have the metabolic activity and the proliferative capacity. The "senescent cells" mean cellular senescence-induced cells which maintain the viable state and have the metabolic activity but which have lost the proliferative capacity. Inhibition of the change into senescent cells suppresses an increase in senescent cells and suppresses accumulation of senescent cells, and as a result, prevention or suppression of various symptoms of aging or delay in the progress thereof can be achieved.

Moreover, the anti-aging effect that the composition of the invention exhibits may be through an improving effect on mitochondrial function. When there is abnormality in mitochondrial function, reactive oxygen species (ROS) are produced excessively, damage cells and promote aging. Therefore, when the mitochondrial function is improved, an anti-aging effect can be achieved. Here, the "improvement of function" includes enhancement of function and also includes prevention, suppression or delay of a decline in function. The mitochondrial function can be generally determined using the ROS production amount as an indicator. Moreover, the decline in function also includes abnormal function.

The anti-aging effect that the composition of the invention exhibits may be through an antioxidative stress-enhancing effect. The oxidative stress in the present specification refers to an impairing effect on a cell or on an individual caused by reactive oxygen species such as free radicals. The antioxidative stress-enhancing effect means enhancement of the resistance of a cell or an individual to oxidative stress. Here, the "enhancement" generally refers to enhancement of antioxidative stress but may include prevention, suppression or delay of a decline in antioxidative stress. The resistance to oxidative stress can be determined using the expression level of a gene relevant to repair of damage by reactive oxygen species (an oxidative stress resistance gene) as an indicator. In the present specification, the antioxidative stress and the resistance to oxidative stress are synonyms.

For example, in a cell which is damaged by reactive oxygen species, an action of repairing the damage is generally exerted, but insufficient repair promotes cellular senescence. Therefore, when the function of repairing the damage by oxidative stress, namely the resistance to oxidative stress, is enhanced, an anti-aging effect can be achieved.

For example, when the resistance to damage by reactive oxygen species which are excessively produced by abnormality in mitochondrial function is enhanced, an anti-aging effect can be achieved.

The composition of the invention exhibits an anti-aging effect as described above and thus can be preferably used for prolonging lifespan or suppressing a decline in motor function. Here, the prolongment of lifespan may include extension of healthy lifespan in addition to simply living long.

The composition of the invention can inhibit induction from normal cells into senescent cells and suppress accumulation of senescent cells as described above and is thus expected to improve or prevent a disease or a symptom which is related to or caused by cellular senescence or SASP secreted from senescent cells. Such diseases and symptoms include premature senescence, cataract, alopecia, cardiomegaly, a fatty liver, interstitial pneumonia, COPD, glomerulosclerosis, arteriosclerosis, arthritis, intervertebral disc degeneration, sarcopenia, inflammatory bowel disease, diabetes, metabolic syndrome, cancers, sarcoma, lymphoma, dementia, pituitary tumor, neurodegenerative diseases and the like. Moreover, with the composition of the invention, effects such as alleviation of a decrease in the bone mass, fat atrophy, improvement of cardiac function, recovery of respiratory function and enhancement of muscles are also expected.

The composition of the invention can improve mitochondrial function as described above and is thus expected to improve or prevent a disease or a symptom which is related to or caused by abnormality in mitochondrial function. Such diseases and symptoms include mitochondrial diseases, musculoskeletal disorders, skin diseases, diabetes, metabolic diseases, neurodegenerative diseases, eye diseases, atherosclerosis, cardiovascular diseases, heart diseases, kidney diseases and the like.

The composition of the invention has an antioxidative stress-enhancing effect as described above and is thus expected to improve or prevent a disease or a symptom caused by oxidative stress. Such diseases and symptoms include hypertension, inflammation, arteriosclerosis, symptoms of aging such as wrinkles and sag, cancers, cranial nerve disease such as Alzheimer's disease, respiratory diseases such as asthma, cataract, heart diseases, gastrointestinal diseases such as a fatty liver, symptoms such as poor concentration and lassitude and the like.

The amount of *Bifidobacterium longum* NITE BP-02621, the culture of the bacterium or the treated product of the bacterium comprised in the composition of the invention is not particularly limited but is, for example, preferably 1.0×10⁴ cfu or more per 1 mL of the composition as the bacterial amount of the bacterium, and more preferably, bacterial cells of 2.0×10⁷ cfu or more may be comprised. In this regard, the cfu refers to colony forming unit. In the present specification, for example, a value obtained by culturing on a solid medium comprising 10 mass% reduced skim milk powder at 38°C can be used. When the bacterial cells are dead cells, the cfu can be replaced with the cells.

When a culture supernatant is used as the culture of the bacterium, the amount is preferably 0.1 to 100 mass% of the entire composition, more preferably 1 to 90 mass%, further preferably 10 to 80 mass%.

The ranges may be generally ranges of amount for distribution as an oral composition.

Another aspect of the invention is a composition comprising one, two or more kinds selected from *Bifidobacterium longum* NITE BP-02621, a culture of the bacterium and a treated product of the bacterium which is used for anti-aging.

Another aspect of the invention is use of one, two or more kinds selected from *Bifidobacterium longum* NITE BP-02621, a culture of the bacterium and a treated product of the bacterium in the manufacture of an anti-aging composition.

Another aspect of the invention is use of one, two or more kinds selected from *Bifidobacterium longum* NITE BP-02621, a culture of the bacterium and a treated product of the bacterium in anti-aging.

Another aspect of the invention is one, two or more kinds selected from *Bifidobacterium longum* NITE BP-02621, a culture of the bacterium and a treated product of the bacterium which are used for anti-aging.

Another aspect of the invention is an anti-aging method including administering one, two or more kinds selected from *Bifidobacterium longum* NITE BP-02621, a culture of the bacterium and a treated product of the bacterium to a subject.

The subject to which the composition of the invention is administered (subject of administration) and the subject which takes the composition (subject of intake) are not particularly limited as long as the subjects are an animal but are generally a human. Moreover, the subjects may be any of an adult, a child, an infant, a newborn (including an underweight infant) and the like but are generally an adult. The gender thereof is not particularly limited.

In the present specification, "administering one, two or more kinds selected from *Bifidobacterium longum* NITE BP-02621, a culture of the bacterium and a treated product of the bacterium to an animal" may be synonymous with "causing an animal to take one, two or more kinds selected from *Bifidobacterium longum* NITE BP-02621, a culture of the bacterium and a treated product of the bacterium". The intake is generally voluntary (free intake) but may be forced (forced intake). That is, the administration step may be specifically, for example, a step of blending one, two or more kinds selected from *Bifidobacterium longum* NITE BP-02621, a culture of the bacterium and a treated product of the bacterium in a food, a drink or feed for supplying to the subject and thus causing the subject to freely take the food, the drink or the feed.

The timing of intake (administration) of the composition of the invention is not particularly limited and can be appropriately selected depending on the condition of the subject of intake (administration).

The intake (dosage) of the composition of the invention is appropriately selected based on the age of the subject of intake (administration), the gender, the condition, other conditions and the like.

The intake (dosage) of the composition of the invention, as the intake (dosage) of the bacterial cells of *Bifidobacterium longum* NITE BP-02621 according to the invention, is, for example, preferably in the range of 0.5 to 5 g/day for an adult, more preferably in the range of 1 to 4 g/day, further preferably 2 to 3 g/day. Alternatively, the intake (dosage) of a culture supernatant of *Bifidobacterium longum* NITE BP-02621 is, for example, preferably in the range of 0.5 to 5 g/day for an adult, more preferably in the range of 1 to 4 g/day, further preferably 2 to 3 g/day.

Regardless of the amount or the period of intake (administration), the composition of the invention can be taken (administered) once a day or in multiple divided portions.

The period of intake (administration) of the composition of the invention is not particularly limited. The upper limit of the period of intake (administration) is not particularly set, and continuous long-term intake (administration) is possible.

The route of intake (administration) of the composition of the invention may be an oral or parenteral route but is preferably an oral route. The parenteral intake (administration) is transdermal, intravenous or rectal administration, inhalation or the like.

When the composition of the invention is an orally taken (administered) composition, the composition is preferably a food or a drink in an embodiment.

The form and the property of the food or the drink are not particularly restricted as long as the food or the drink does not impair the effects of the invention and can be orally taken (administered), and the food or the drink can be produced by a general method using a material which is generally used for a food or a drink except that one, two or more kinds selected from *Bifidobacterium longum* NITE BP-02621, a culture of the bacterium and a treated product of the bacterium are comprised.

The food or the drink is not limited regarding the form such as liquid, paste, gel solid or powder. Examples include the following examples: nutritional supplements (supplements), tablet candies; liquid foods (nutrition products for tube feeding); wheat products such as breads, macaroni, spaghetti, noodles, cake mixes, frying flours and bread crumbs; instant foods such as instant noodles, cup noodles, retort-pouched/prepared foods, prepared canned foods, microwave foods, instant soups/stews, instant miso soups/clear Japanese soups, canned soups, freeze-dried foods and other instant foods; processed agricultural products such as canned agricultural products, canned fruits, jams/marmalades, pickles, cooked beans, dried agricultural products and cereals (processed grains); processed fishery products such as canned fishery products, fish hams/sausages, fishery paste products, fishery delicacies and *Tsukudani* (foods boiled down in sweetened soy sauce); processed livestock products such as canned livestock products/pastes and livestock hams/sausages; milk/dairy products such as processed milk, milk beverages, yogurts, lactic acid bacteria beverages, cheeses, ice creams, powdered formula, creams and other dairy products; oils and fats such as butter, margarine and vegetable oils; basic condiments such as soy sauce, soybean paste, sauces, processed tomato condiments, *Mirin* (sweet sake for seasoning) and vinegars; compound flavor enhancers/foods such as cooking mixes, curry roux, sauces, dressings, noodle broths, spices and other compound flavor enhancers; frozen foods such as frozen food materials, semi-cooked frozen foods and cooked frozen foods; confectioneries such as caramels, candies, chewing gums, chocolates, cookies, biscuits, cakes, pies, snacks, crackers, Japanese-style confectioneries, rice confectioneries, bean confectioneries, desserts, jellies and other confectioneries; luxury beverages such as carbonated drinks, natural juices, fruit juices, fruit juice-comprising soft drinks, fruit flesh drinks, fruit granule-comprising fruit juices, vegetable drinks, soy milk, soy milk drinks, coffee drinks, tea drinks, drink powders, concentrated drinks, sport drinks, nutritional drinks, alcohols and other luxury beverages, other commercial foods such as baby foods, *Furikake* (dry Japanese seasonings) and seasonings for *Chazuke* (boiled rice with hot tea) and the like; powdered infant formula; enteral nutrition products; functional foods (foods for specified health uses and foods with nutrient function claims); and the like.

An embodiment of the food or the drink can be feed. The feed is pet food, livestock feed, fish farming feed or the like.

The form of the feed is not particularly restricted, and the feed may contain, in addition to one, two or more kinds selected from *Bifidobacterium longum* NITE BP-02621, a culture of the bacterium and a treated product of the bacterium, for example: grain such as corn, wheat, barley, rye and milo; vegetable oil cake such as soybean oil cake, rapeseed oil cake, coconut oil cake and linseed oil cake; bran such as oat bran, wheat bran, rice bran and defatted rice bran; a food manufacturer's by-product such as corn gluten meal and corn jam meal; animal feed such as fish powder, skim milk powder, casein, yellow grease and tallow; yeast such as torula yeast and brewer's yeast; mineral feed such as tertiary calcium phosphate and calcium carbonate; an oil or a fat; a single amino acid; a saccharide; or the like.

When the composition of the invention is an embodiment of a food or a drink (including feed), the composition can be provided/sold as a food or a drink labeled with use related to anti-aging.

The "labeling" act includes all the acts for informing a consumer of the use, and all the expressions which can remind of/cause to guess the use are the "labeling" acts of the invention, regardless of the purposes of labeling, the contents of labeling, the objects to be labeled, the media and the like.

The "label" is preferably with an expression which allows a consumer to directly recognize the use. Specific examples include an act of transferring an article in which the use is described on a product regarding the food or the drink or packaging of a product, delivering such an article, displaying such an article for transfer or delivery or importing such an article, an act of displaying or distributing an advertisement of a product, a price list or a business document with a description of the use thereon or providing information with such contents with a description of the use by an electromagnetic method (internet or the like) and another act.

The content of the label is preferably a label approved by the administration or the like (for example, a label approved based on a system provided by the administration and provided in the form based on the approval or the like). It is preferable to label with such a content on packaging, a container, a catalogue, a brochure, an advertisement material in a sales site such as POP, other documents or the like.

The "labels" also include labels with health foods, functional foods, enteral nutrition products, food for special dietary uses, food with health claims, foods for specified health uses, foods with nutrient function claims, foods with function claims, quasi-drugs and the like. In particular, the labels are labels approved by the Consumer Affairs Agency, such as labels approved by the systems for foods for specified health uses, foods with nutrient function claims or foods with function claims and labels approved by a similar system. Specific examples include a label with foods for specified health uses, a label with qualified foods for specified health uses, a label indicating influence on the structure or the function of a body, a label with reduction of disease risk, a label with a scientifically grounded function and the like. More specifically, typical examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009) and similar labels.

The labels are, for example, labels with "for those who wish to prevent aging", "for improving motor function", "for those who wish to prolong healthy lifespan", "for those who wish to stay young", "for those who wish to enhance antioxidative capability", "enhance antioxidative capability", "for those who wish to enhance resistance to oxidative stress" and the like.

In an embodiment, the composition of the invention can be a pharmaceutical product.

The intake (administration) route of the pharmaceutical product may be an oral or parenteral route but is preferably an oral route. The parenteral intake (administration) is transdermal, intravenous or rectal administration, inhalation or the like.

Regarding the form of the pharmaceutical product, the composition can be appropriately formulated into a desired dosage form depending on the intake (administration) method. For example, in the case of oral intake (administration), the composition can be formulated into a solid preparation such as powder, granules, tablets and capsules or the like, a liquid preparation such as a solution, a syrup, a suspension and an emulsion or the like. In the case of parenteral intake (administration), the composition can be formulated into a suppository, ointment, an injection or the like.

For the formulation, a component which is generally used for formulation such as excipients, pH-adjusting agents, colorants and corrigents can be used. Another medicinal component or another medicine such as a component having an anti-aging effect which is known or will be found in the future can also be used in combination.

In addition, the formulation can be appropriately conducted by a known method depending on the dosage form. For the formulation, a carrier which is generally used for formulation may be appropriately blended and formulated. Such carriers are excipients, binders, disintegrating agents, lubricants, stabilizers, flavoring agents and the like.

Examples of the excipients include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; sulfate derivatives such as calcium sulfate; and the like.

Examples of the binders include, in addition to the excipients, gelatin, polyvinylpyrrolidone, macrogol and the like.

Examples of the disintegrating agents include, in addition to the excipients, chemically modified starch or cellulose derivatives such as croscarmellose sodium, sodium carboxymethyl starch and cross-linked polyvinylpyrrolidone and the like.

Examples of the lubricants include: talc; stearic acid; metal stearates such as calcium stearate and magnesium stearate; colloidal silica; waxes such as veegum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; starch derivatives; and the like.

Examples of the stabilizers include: paraoxybenzoate esters such as methylparaben and propylparaben; alcohols such as chlorobutanol, benzyl alcohol and phenylethyl alcohol; benzalkonium chloride; acetic anhydride; sorbic acid; and the like.

Examples of the flavoring agents include sweeteners, acidulants, aromas and the like.

In this regard, the carriers used in the case of a liquid preparation for oral intake (administration) include solvents such as water and the like.

The timing for taking (administering) the pharmaceutical product of the invention is not particularly limited, and examples include before a meal, after a meal, between meals, before bedtime and the like.

### Examples

The invention is explained further specifically below using Examples, but the invention is not limited to the Examples.

### [Example 1] Preparation of Culture Supernatant of Bifidobacterium longum NITE BP-02621

*Bifidobacterium longum* NITE BP-02621 (BB536) was precultured in MRS medium (Difco Lactobacillo MRS Broth, manufactured by Becton, Dickinson and Company). The preculture solution was inoculated to GAM medium (GAM broth "Nissui", manufactured by Nissui Pharmaceutical Co., Ltd.) at 5% (v/v) and cultured at 37°C for 16 hours. After the culture solution was centrifuged for 10 minutes under the conditions of 4°C and 8000 G to collect the supernatant, the pH was adjusted to 7.0 ± 0.1, and a culture supernatant was thus prepared by filtering through a filter of 0.22 µm. The culture supernatant was stored at -30°C.

The obtained "culture supernatant of *Bifidobacterium longum* NITE BP-02621 (BB536)" is also simply called the "culture supernatant of BB536" below.

### [Test Example 1] Examination of Cellular Senescence Inhibitory Effect

To a 6-well plate (Falcon cell culture 6-well multiwell plate, manufactured by Corning), MEM medium (Minimum Essential Medium Eagle With Earle's salts, manufactured by SIGMA), 10% FBS (Fetal Bovine Serum, manufactured by MP Bio) and 1% P/S (Pen Strep, manufactured by gibco) were added each in a volume of 2 mL, and TIG-3 cells (1801781/07262017/JCRB Cell Bank) having a PDL (Population Doubling Level) value of 25 to 50 were inoculated at 1×10⁴ cells/well. The culture supernatant of BB536 prepared in Example 1 was added thereto at 1% (v/v) or 0.1% (v/v), and the cells were cultured under the conditions of 37°C and 5% CO₂.

Doxorubicin (Dox; doxorubicin hydrochloride, manufactured by FUJIFILM Wako Pure Chemical Corporation) at a final concentration of 3 µM was added 18 hours after starting the culture, and senescence-inducing stimulation was conducted for an hour. After the senescence-inducing stimulation, the cells were washed twice with 2 mL of phosphate-buffered saline (PBS pH 7.4 (1X), manufactured by gibco), and 2 mL of MEM medium, 10% FBS and 1% P/S were added. The MEM medium, FBS and P/S used were the same as those used for inoculating TIG-3 cells.

Then, the culture supernatant of BB536 was added again at 1% (v/v) or 0.1% (v/v), and the cells were cultured for two to three days. The obtained samples are called "BB536 culture supernatant groups" below.

In this regard, wells to which the culture supernatant of BB536 was not added were also provided (Dox group). Moreover, as a control for Dox, wells to which Dox and the culture supernatant of BB536 were not added and to which DMSO was added were also provided (DMSO group) . In addition, wells to which 1 µL of 1M N-acetylcysteine (manufactured by Nacalai Tesque, Inc.), which is a known antioxidant, was added instead of adding the culture supernatant of BB536 (NAC group) and wells to which GAM medium was added instead of adding the culture supernatant of BB536 (GAM group) were provided as a positive control for the BB536 culture supernatant groups and a negative control for the BB536 culture supernatant groups, respectively.

After 48-hour culture after the second addition of the culture supernatant of BB536, senescence-associated β-galactosidase (SA-β-Gal) staining, which is for specifically staining senescent cells, was conducted. Pictures of the SA-β-Gal-stained cells were taken at three sites using a bright-field microscope (5× objective lens), and the cell numbers were counted as senescent cells.

Moreover, after 72-hour culture after the second addition of the culture supernatant of BB536, the expression of *p16* gene, which is a senescent cell marker, was analyzed.

The reverse transcription was conducted using PrimeScript RT Reagent Kit (manufactured by Takara Bio Inc.), and qPCR was conducted using TB Green Premix Ex Taq II (manufactured by Takara Bio Inc.).

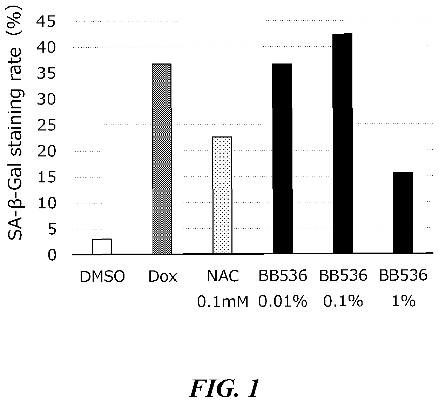

The percentages of the SA-β-Gal-stained cells relative to the total inoculated cells (staining rates %) of the groups are shown in Fig. 1. While the SA-β-Gal-positive cells accounted for 36% in the Dox group, the SA-β-Gal-positive cells were 15% in the BB536 culture supernatant group (1%). The results of the BB536 culture supernatant group are results showing that the staining rate thereof was lower than the staining rate of the SA-β-Gal-positive cells (23%) of the NAC group, which is a positive control.

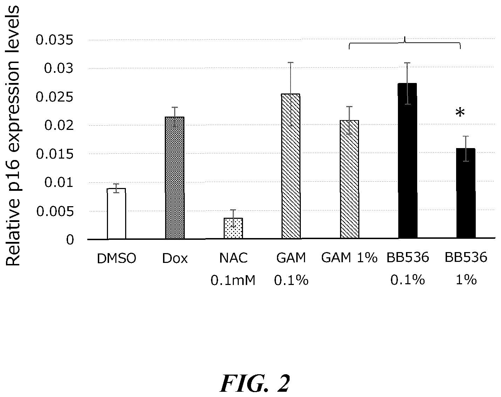

The expression levels of *p16* gene (the relative expression levels to that of GAPDH as an internal control) of the groups are shown in Fig. 2. While the relative expression level of the Dox group was 0.0214, that of the 1% BB536 culture supernatant-administered group was 0.0156, which was significantly lower than that of the Dox group (Welch's t-test, p value of 0.0387).

From the above results, it was found that the culture supernatant of *Bifidobacterium longum* NITE BP-02621 has a cellular senescence inhibitory effect.

### [Example 2] Preparation of Bacterial Cells of Bifidobacterium longum NITE BP-02621

*Bifidobacterium longum* NITE BP-02621 (BB536) was cultured using MRS medium (Difco Lactobacillo MRS Broth, manufactured by Becton, Dickinson and Company) under anaerobic conditions at 37°C. After 16 hours of culture, the bacterial cells were collected by centrifugation.

The obtained "bacterial cells of *Bifidobacterium longum* NITE BP-02621 (BB536)" are also simply called "bacterial cells of BB536" below.

### [Test Example 2] Examination of Lifespan-Prolonging Effect in Nematodes

### (1) Nematode Culture

Nematodes (*Caenorhabditis elegans,* strain N2) and *Escherichia coli* OP50 (OP50 below) as the feed thereof were obtained from CAENORHABDITIS GENETICS CENTER.

OP50 was cultured under aerobic conditions at 37°C using LB medium (manufactured by Becton, Dickinson and Company). After 16 hours of culture, the bacterial cells of OP50 were collected by centrifugation. The obtained bacterial cells of OP50 were smeared on an NGM plate (OP50 bacterial cells 300 mg/plate) and used for culturing nematodes. Alternatively, the bacterial cells of OP50 were added to S-complete medium (OP50 bacterial cells 6 mg/mL). The other procedures regarding the reagents, nematode culture and synchronization were conducted in accordance with the reference documents below.

### Reference Documents

S. Brenner, Genetics, 77 (1), 71-94, 1974, The Genetics of Caenorhabditis Elegans
F. Amrit, et. Al., Methods,68 (3), 465-475, 2014, The C. elegans lifespan assay toolkit

### (2) Preparation of Test Plates

A control plate was prepared by adding 2'-deoxy-5-fluorouridine (manufactured by Tokyo Chemical Industry Co., Ltd.) to the OP50-smeared NGM plate (control group). The addition of 2'-deoxy-5-fluorouridine is to prevent attachment of new individuals. Regarding the control plate, a half of the amount of normal OP50 feed was replaced with the bacterial cells of BB536 collected by the above method (BB536 bacterial cell-administered group).

### (3) Assessment Method of Lifespan

Synchronized nematodes (L4/adult period) were cultured in 2'-deoxy-5-fluorouridine-comprising S-complete medium. Normal OP50 feed was given to the control group (n=127), and feed obtained by replacing a half of the amount of normal OP50 feed with the bacterial cells of BB536 was given to the BB536 bacterial cell-administered group (n=118). The numbers of living individuals were counted using a microscope every two to three days. The survival curves were calculated from the obtained data using the Kaplan-Meier method.

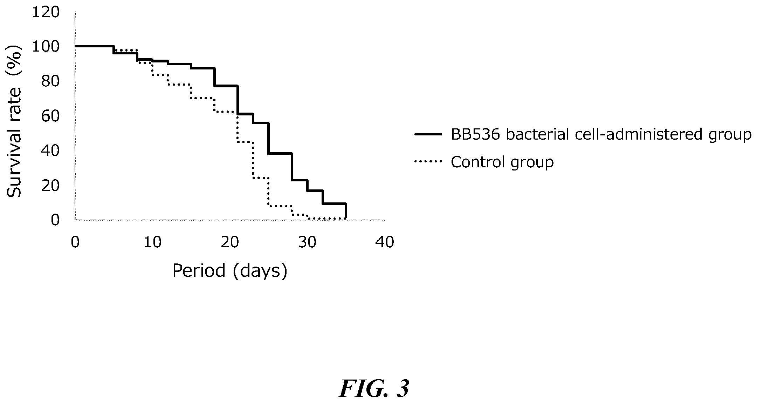

The survival curves (lifespan) in which the third day after hatching (L4/adult period) was considered as day 0 are shown in Fig. 3. A significant lifespan-prolonging effect was observed in the BB536 bacterial cell-administered group. Specifically, while the average lifespan (days) of the control group was 19.4 ± 0.57, that of the BB536 bacterial cell-administered group was 23.7 ± 0.69.

### [Test Example 3] Examination of Motor Function-Improving Effect in Aged Nematodes

The nematode culture and the preparation of test plates were conducted in the same manners as those in Test Example 2.

Aged nematodes cultured for 14 days on the control plate or the test product plate were picked with platinum loops and moved into M9 buffer, and the numbers of crawling movements were counted for 30 seconds under a microscope. The measurement was made per individual.

The numbers of 30-second crawling movements of nematodes in the aged period are shown in Table 1. It was observed that the decline in motor function declined with age was suppressed with BB536. Specific numbers were 24.8 ± 3.3 in the control group and 33.5 ± 4.0 in the BB536 bacterial cell-administered group.

### [Table 1]

**(Table 1) Numbers of Crawling Movements of Nematodes in Aged Period**

| | Number of Crawling |
|---|---|
| | Movements (Number) |
| | Average ± Standard Error |
| Control Group (n=14) | 24.8 ± 3.3 |
| BB536 Bacterial Cell-Administered Group (n=16) | 33.5 ± 4.0 |
| The values are numbers of the crawling movements in 30 seconds. | |

### [Test Example 4] Examination of Improvement Effect on Mitochondrial Dysfunction

The nematode culture and the preparation of test plates were conducted in the same manners as those in Test Example 2.

Nematodes which were cultured for seven days on the control plate or the test product plate were suspended in M9 buffer, inoculated to a 96-well plate and cultured for six hours after adding paraquat (final concentration: 1 mM) to induce mitochondrial dysfunction and H2DCFDA (final concentration 500 µM). Then, the production amounts of reactive oxygen species (ROS) were quantified by measuring the fluorescence intensities (Ex: 480 nm/Em :530 nm) using a plate reader, and the nematode number in each well was counted under a microscope and used for correction.

The ROS production amounts of the nematodes are shown in Table 2. Suppression of ROS production by BB536 was observed. Specifically, it was observed that the ROS production was suppressed to 0.40 times in the BB536 bacterial cell-administered group as compared to the control.

### [Table 2]

**(Table 2) ROS Production Amounts of Nematodes**

| | Paraquat(-) | Paraquat(+) |
|---|---|---|
| Control Group (n=2) | 9997 | 72442 |
| BB536 Bacterial Cell-Administered Group (n=2) | 7936 | 29116 |
| The values are fluorescence intensity/individual number. | | |

### [Test Example 5] Examination of Resistance to Mitochondrial Dysfunction

The nematode culture and the preparation of test plates were conducted in the same manners as those in Test Example 2.

Synchronized nematodes (L4/adult period) were cultured in 2'-deoxy-5-fluorouridine-comprising S-complete medium. Paraquat (final concentration: 10 mM) was added to the medium to induce mitochondrial dysfunction. Normal OP50 feed was given to the control group (n=58), and feed obtained by replacing a half of the amount of normal OP50 feed with the bacterial cells of BB536 was given to the BB536 bacterial cell-administered group (n=43). The numbers of living individuals were counted using a microscope every two to three days. The survival curves were calculated from the obtained data using the Kaplan-Meier method.

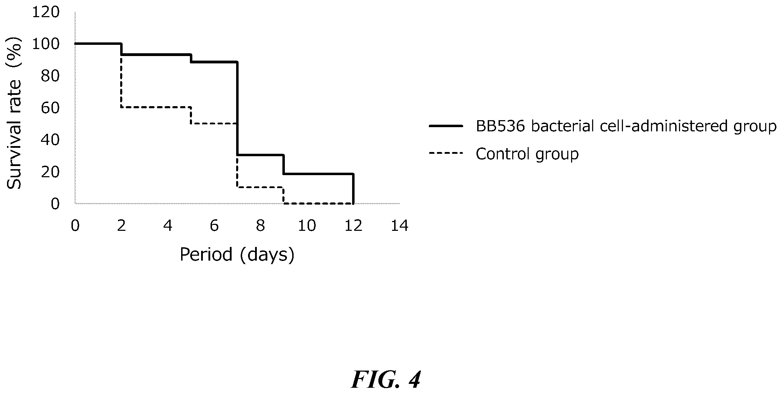

The survival curves (lifespan) in which the third day after hatching (L4/adult period) was considered as day 0 are shown in Fig. 4. A significant lifespan-prolonging effect was observed in the BB536 bacterial cell-administered group. Specifically, while the average lifespan (days) of the control group was 5.01 ± 0.34, that of the BB536 bacterial cell-administered group was 7.16 ± 0.27.

### [Test Example 6] Examination of Oxidative Stress Resistance Gene Expression-Promoting Effect

The nematode culture and the preparation of test plates were conducted in the same manners as those in Test Example 2.

Aged nematodes (n=6 for each group) which were cultured for 14 days on the control plate or the test product plate were collected, and after the total RNA was collected using RNeasy Mini kit (QIAGEN), cDNA was produced using TaKaRa PrimeScript (registered trademark) RT reagent Kit. The expression levels of heat shock protein 70 *(HSP70),* superoxide dismutase 1 (*SOD-1*)*,* thioredoxin-1 (trx-1) and glutathione S-transferase 4 (*gst-4*), which are oxidative stress resistance genes, and the expression level of *ACT-1* as an internal control gene were quantitatively analyzed using the cDNA as a sample by the real-time PCR method. The expression levels of the oxidative stress resistance genes were corrected using the expression levels of *ACT-1.*

The expression levels of the oxidative stress resistance genes in nematodes are shown in Table 3. It was observed that the expression levels of *HSP70, SOD-1, trx-1* and *gst-4,* which are oxidative stress resistance genes, were increased by BB536.

### [Table 3]

**(Table 3) Expression Levels of Oxidative Stress Resistance Genes in Nematodes**

| | Control Group (n=6) | BB536 Bacterial Cell-Administered Group (n=6) |
|---|---|---|
| HSP70 | 1.00 ± 0.14 | 1.85 ± 0.38 |
| SOD-1 | 1.00 ± 0.15 | 1.75 ± 0.40 |
| trx-1 | 1.00 ± 0.10 | 1.44 ± 0.22 |
| gst-4 | 1.00 ± 0.15 | 1.95 ± 0.46 |

From the results of Test Examples 2 to 6, it was shown that BB536 has a lifespan-prolonging effect through aging suppression. The mechanisms suggested therefor are an effect of suppressing abnormality of mitochondrial function and an antioxidative stress-enhancing effect.

## Claims

1. An anti-aging composition comprising one, two or more kinds selected from *Bifidobacterium longum* NITE BP-02621, a culture of the bacterium and a treated product of the bacterium.

2. The composition according to claim 1 from which an anti-aging effect is obtained through a cellular senescence inhibitory effect and/or a mitochondrial function-improving effect.

3. The composition according to claim 1 or 2 which is used for prolonging lifespan and/or for suppressing a decline in motor function.

4. A composition comprising one, two or more kinds selected from *Bifidobacterium longum* NITE BP-02621, a culture of the bacterium and a treated product of the bacterium which is used for anti-aging.

5. The composition according to any one of claims 1 to 5 which is a food or a drink.

6. The composition according to any one of claims 1 to 5 which is a pharmaceutical product.

7. Use of one, two or more kinds selected from *Bifidobacterium longum* NITE BP-02621, a culture of the bacterium and a treated product of the bacterium in the manufacture of an anti-aging composition.

8. Use of one, two or more kinds selected from *Bifidobacterium longum* NITE BP-02621, a culture of the bacterium and a treated product of the bacterium in anti-aging.

9. An anti-aging method including administering one, two or more kinds selected from *Bifidobacterium longum* NITE BP-02621, a culture of the bacterium and a treated product of the bacterium to a subject.
